(19) Europäisches Patentamt
European Patent Office
Office européen des brevets

(11) **EP 1 853 925 B1**

(12) **EUROPÄISCHE PATENTSCHRIFT**

(45) Veröffentlichungstag und Bekanntmachung des Hinweises auf die Patenterteilung:
**28.12.2011 Patentblatt 2011/52**

(21) Anmeldenummer: **06707077.1**

(22) Anmeldetag: **17.02.2006**

(51) Int Cl.:
*G01N 33/86* (2006.01)

(86) Internationale Anmeldenummer:
**PCT/EP2006/001492**

(87) Internationale Veröffentlichungsnummer:
**WO 2006/089697 (31.08.2006 Gazette 2006/35)**

(54) **VERFAHREN ZUR BESTIMMUNG DER GESAMTGERINNUNGSAKTIVITÄT EINER BLUT- ODER PLASMAPROBE**

METHOD FOR DETERMINING THE TOTAL COAGULATION ACTIVITY OF A BLOOD OR PLASMA SAMPLE

PROCEDE POUR DETERMINER L'ACTIVITE DE COAGULATION GLOBALE D'UN ECHANTILLON DE SANG OU DE PLASMA

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **22.02.2005 DE 102005008066**

(43) Veröffentlichungstag der Anmeldung:
**14.11.2007 Patentblatt 2007/46**

(73) Patentinhaber: **JenAffin GmbH**
**07745 Jena (DE)**

(72) Erfinder:
• **NOWAK, Götz**
**99097 Erfurt (DE)**
• **BUCHA, Elke**
**99094 Erfurt (DE)**
• **LANGE, Ute**
**07768 Kahla (DE)**

(74) Vertreter: **Hiebl, Inge Elisabeth et al**
**Kraus & Weisert**
**Patent- und Rechtsanwälte**
**Thomas-Wimmer-Ring 15**
**80539 München (DE)**

(56) Entgegenhaltungen:
**EP-A- 1 367 135**   **US-A- 6 051 390**
**US-A1- 2004 043 428**   **US-B1- 6 743 596**

• **MARKWARDT F: "[Quantitative determination of prothrombin by titration with hirudin.]" NAUNYN-SCHMIEDEBERGS ARCHIV FÜR EXPERIMENTELLE PATHOLOGIE UND PHARMAKOLOGIE. 1958, Bd. 232, Nr. 3, 1958, Seiten 487-498, XP009067800**
• **LANGE U ET AL: "Ecarin chromogenic assay - A new method for quantitative determination of direct thrombin inhibitors like hirudin" PATHOPHYSIOLOGY OF HAEMOSTASIS AND THROMBOSIS, Bd. 33, Nr. 4, 2003, Seiten 184-191, XP002385856 ISSN: 1424-8832**

**Beschreibung**

Die Erfindung betrifft ein Verfahren zur Bestimmung der Gesamtgerinnungsaktivität einer Blut- oder Plasmaprobe. Die Erfindung betrifft ferner ein Kit zur Durchführung dieses Verfahrens sowie das Kit zur Verwendung in einem Verfahren zum Erhalt von Aussagen über den Gerinnungsstatus eines Patienten.

Die Blutgerinnung in einem menschlichen oder tierischen Organismus ist ein komplexer, in Phasen ablaufender Vorgang, der durch pathologische und physiologische Prozesse ausgelöst wird und in vivo der Blutstillung dient. Dabei erfolgt die Umwandlung des im Plasma vorhandenen löslichen Fibrinogens in den fasrig-galertigen Gerinnungsstoff, das Fibrin, in einem mehrstufigen Prozess (Gerinnungskaskade), an dem mindestens 15 verschiedene Blutgerinnungsfaktoren beteiligt sind, von denen jeder, wenn er aktiviert ist, jeweils die nächste inaktive Vorstufe aktiviert.

Die Serinproteinase Thrombin ist das wichtigste Enzym während der Aktivierung des plasmatischen Gerinnungssystems, bei dem durch in mehreren Teilschritten generierte Aktivierungskomplexe aus Vorstufen von Serinproteinasen, die über spezielle Bindungsstrukturen ($\gamma$-Carboxygruppen) an negative geladene Phospholipide durch Vermittlung von Calciumionen und Co-Faktoren (FVa, FVIIIa) fixiert sind, quasi festphasenartig Serinproteinasen (FXI, FIX, FX) aktiviert werden. Das letzte Produkt der komplexen Gerinnungsaktivierung, das Enzym Thrombin, verlässt als einzige Protease seinen Aktivierungskomplex und ist somit in freier Form in der Blutzirkulation verfügbar. Dort trifft es auf sehr unterschiedliche Thrombinsubstrate, die sowohl das Gerinnungsprotein Fibrinogen als auch Zellen betreffen. Thrombin hat dadurch eine Brückenfunktion zwischen plasmatischer Gerinnung und den zellulären Elementen, vor allem den Blutplättchen, für die Thrombin der stärkste aggregationsauslösende Faktor darstellt. Auf der Phospholipidoberfläche von aggregierten Plättchen befinden sich ebenfalls große Mengen von Aktivierungskomplexen, die dann Thrombin generieren können. An der maximal möglichen Thrombinmenge in Blut und Plasma lässt sich der aktuelle Gerinnungsstatus ("global coagulation state") eines Patienten ablesen. Dieses sogenannten Thrombingenerierungspotenzial erlaubt Hinweise für eine Übergerinnbarkeit des Blutes (Thrombophilie), kann aber auch eine Unterfunktion der Thrombinbildung (Hämophilie, Blutungstendenz) erkennen lassen.

Im Stand Technik sind Methoden zur Messung der Thrombingenerierung in Plasma bekannt. Dies erfolgte im Allgemeinen mit Hilfe von globalen Gerinnungstests, wie zum Beispiel aPTT, Prothrombinzeit, Quickwert, Reptilasezeit, Batroxobinzeit etc. Mit derartigen Tests wird aber nur ein Ausschnitt der Gerinnungsaktivierung erfasst. Grundsätzlich wird hierbei in vitro vor allem in Plasmaasservaten von Patienten eine bestimmte Menge eines Gerinnungsinduktors zugesetzt. Es wird dadurch eine gewisse Menge von $\alpha$-Thrombin-Aktivität aktiviert, die ausreicht, um über ein Erkennungssystem, in der Regel das in der Probe vorhandene Fibrinogen, den Eintritt der Gerinnung zeitlich zu erfassen. Dabei werden im Prinzip nur pro ml Gerinnungsansatz 5-8 NIH-Einheiten Thrombin aktiviert.

Eine Methode zur Messung der Thrombingenerierung in Plasma ist das Verfahren nach Hemker et al. (EP 0 420 332). Mit dieser in mehreren Modifikation in der Praxis eingesetzten Hemker-Methode wird die Thrombingenerierung nach Gerinnungsinduktion mittels chromogenen Substrats kontinuierlich messend verfolgt (WO 0052199, WO 03093831, WO 09621740). Dabei wird in kurzen Zeitabständen die chromogene Substratspaltung als Flächenintegral aufaddiert. Die Geschwindigkeit der chromogenen Substratspaltung sowie die chromogene Substratspaltung über die Zeit werden als Maß der Thrombingenerierung herangezogen. Mit dieser Methode können Einflüsse von gerinnungshemmenden Pharmaka sowie Gerinnungsstörungen durch die simultane Thrombingenerierung messbar gemacht werden. Der Nachteil dieses Verfahrens besteht jedoch darin, dass aufgrund des Messprinzips mittels chromogener bzw. fluorgoener Substrate und photometrischer Verfahren nur in lichtdurchlässigen Materialien (Plasma bzw. plättchenreiches Plasma) gearbeitet werden kann. Gleichzeitig liegt ein weiterer Nachteil darin, dass die Plasmen zur Messung stark verdünnt werden müssen, was die Einträge der natürlich vorhandenen Inhibitoren verfälscht. Weiterhin kommt es bei derartigen Versuchsansätzen zu einer unphysiologischen Anreicherung von Thrombin durch die permanente Induktion der Gerinnung, so dass dann eine unphysiologische Selbstlimitierung der Aktion durch die im Blut vorhandenen langsam reagierenden Inhibitoren (z.B.: $\alpha_2$-Makroglobulin) stattfindet. Bei in vivo-Situationen sind diese großen freien Thrombinmengen nicht verfügbar, da in unverdünntem Blut eine große Anzahl von Thrombinsubstraten (Fibrinogen, Faktoren V, VIII und XI, Prothrombin), aber auch Antithrombine (Antithrombin II, Heparin-Cofaktor II u.a.) vorhanden sind, die je nach verfügbarer Menge Thrombin dieses effektiv blockieren. Ferner wird bei diesem Verfahren aus dem Stand der Technik nur die dynamische Aktivierung von Thrombin gemessen, es liegt jedoch keine Endpunktmessung der Gerinnungsaktivität vor. Es besteht somit ein Bedarf nach einem zuverlässigen Verfahren zur Messung der maximalen Thrombinaktivierbarkeit, d.h. einer Endpunktmessung der Gerinnungsaktivität.

Die Druckschrift Arch. exper. Path. u. Pharmakol., Bd. 232, S. 487-498 (1958), "Die quantitative Bestimmung des Prothrombins durch Titration mit Hirudin", Franz Markwardt, beschreibt eine Methode zur quantitativen Bestimmung des Prothrombins auf der Grundlage zwischen Hirudin und Thrombin.

EP 1 367 135 beschreibt ein Verfahren zur Bewertung der Thrombinbildung in Blut oder Plasma, welches die initiale Phase der Thrombingenerierung betrifft.

Bei diesem Verfahren wird die Blut oder Plasmaprobe mit mindestens einem Aktivator des Gerinnungssystems und einem Thrombinsubstrat in einer relativ niedrigen Konzentration vermischt und die Freisetzung des Umwandlungsprodukts des Thrombinsubstrats bestimmt.

US 6,051,390 beschreibt die Verwendung eines Thrombininhibitors, der an einen hochmolekularen Träger gebunden ist, als molekularen Marker zur Bestimmung der Gerinnungsaktivierung in der Gerinnungsdiagnostik und der Therapieüberwachung.

Der Erfindung liegt somit die Aufgabe zugrunde, ein Verfahren zur Messung der maximalen Thrombinaktivierbarkeit in Blut oder Plasma bereitzustellen. Das erfindungsgemäße Verfahren soll unabhängig von variablen äußeren Einflüssen, wie zum Beispiel Reaktionstemperatur oder Reaktionszeit, sein und mit ausreichender Sicherheit die Thrombinaktivierung bis zum Endpunkt ablaufen lassen. Ferner soll das Verfahren auch in Vollblut und mit nahezu unverdünnten Blutproben durchführbar sein. Das Verfahren soll ferner einfach anwendbar sein und zuverlässig Auskunft über den Gerinnungsstatus einer Blutprobe erlauben.

Überraschenderweise wurde nun gefunden, dass diese Aufgabe gelöst wird, indem man einen hochspezifischen, nicht-irreversiblen Thrombinhemmstoff, ausgewählt aus Dipetalogastin I, Dipetalogastin II, Rhodniin und Hirudin, in einer bestimmten Menge in der Blutprobe vorlegt und dann nach Gerinnungsinduktion die verbrauchte Menge des zugesetzten Thrombinhemmstoffs bestimmt.

Die Erfindung betrifft somit ein Verfahren zur Bestimmung der Gesamtgerinnungsaktivität einer Blut- oder Plasmaprobe, zur Entscheidung über "gerinnungskrank" oder "gerinnungsgesund", dadurch **gekennzeichnet**, dass man einen hochspezifischen, nicht irreversiblen Thrombinhemmstoff, ausgewählt aus Dipetalogastin I, Dipetalogastin II, Rhodniin und Hirudin, in definierter Menge einer Blut- oder Plasmaprobe zusetzt, die Gerinnung in der Blut- oder Plasmaprobe durch Zusatz von extrinsischen und intrinsischen Faktoren induziert und nach einer definierten Zeit die verbrauchte Menge des zugesetzten Thrombinhemmstoffs in an sich bekannter Weise bestimmt. Ferner betrifft die Erfindung ein Kit zur Durchführung des Verfahrens zur Bestimmung der Gesamtgerinnungsaktivität sowie die Verwendung des Kits zum Erhalt von Aussagen über den Gerinnungsstatus einer Blutprobe.

Es wurde gefunden, dass mit dem erfindungsgemäßen Verfahren in unverdünntem antikoaguliertem Blut und/ oder Plasma die maximal mögliche Thrombingenerierung als Gesamtquantität quantifizierbar ist. Der hochspezifische, nicht-irreversible Thrombinhemmstoff muss in einer relativ hohen Konzentration vorgelegt werden, damit er auch mit den hochaffinen bzw. in relativ hoher Konzentration vorhandenen Thrombinsubstraten und -rezeptoren konkurrenzfähig ist. Die Vorlage von 10 bis 30 $\mu$g hochspezifischer Thrombinhemmstoff pro ml Blut oder Plasma ist in der Regel ausreichend. Das genaue Verhältnis von Hemmstoff zu Blut lässt sich jedoch anhand einfacher Routineversuche leicht experimentell bestimmen.

Wesentlich ist, dass sich bei dem hochspezifischen, nicht-irreversiblen Thrombinhemmstoff um einen Hemmstoff handelt, dessen Affinität sowohl gegen das aktive Zentrum von Thrombin als auch zusätzlich gegen die wichtige Erkennungsstruktur der "anion binding exosite 1" (ABE 1) gerichtet ist. Ferner muss der hochspezifische Thrombininhibitor andere am Gerinnungssystem beteiligte Serinproteinasen der Vorphasen- oder Hauptphasenaktivierung (Faktor VIIa, IXa, Xa, XIa) unbeeinflusst lassen. Erfindungsgemäß werden als Thrombinhemmstoffe Hirudin, Dipetalogastin I und II und Rhodniin aus Rhodnius prolixus (Raubwanze) verwendet.

Vorteilhafterweise sollte der Verbrauch des vorgelegten Hemmstoffs sehr präzise messbar sein. Grundsätzlich können die vorstehend genannten direkten Thrombinhemmstoffe, insbesondere Hemmstoffe mit tight binding-Qualität sehr präzise mit der Ecarin Clotting Time oder in Ableitung davon mit dem Ecarin Chromogenic Assay (WO 0046602) bestimmt und über einen weiten Bereich linear präzise bestimmt werden. Der bevorzugt verwendete Thrombinhemmstoff ist Hirudin. Bevorzugt wird Hirudin in einer Menge von 15 $\mu$g/ml Blut/Plasma verwendet.

Die zu untersuchende Blutprobe/Plasmaprobe kann verdünnt oder unverdünnt verwendet werden. Die Blutprobe bzw. Plasmaprobe wird in an sich bekannter Weise gewonnen. Üblicherweise werden Blutproben in kommerziell erhältlichen fertigen Setups gewonnen. Für das erfindungsgemäße Verfahren werden bevorzugt Sarstedt-Monovetten® 2,7 ml enthaltend 0,3 ml Natrium citricum benutzt, wobei das Blut mittels Venenpunktion der V. cubitalis gewonnen wird. Das Zitratblut wird in 2 aliquote Hälften geteilt und 1 Probe dann anschließend bei 3800 U/min für 10 min in einer Laborzentrifuge behandelt. Das überstehende plättchenfreie Plasma wird mittels Pipette abgenommen und dann nach Vorschrift verwendet. Es ist vorteilhaft, wenn die Untersuchung des Zitratblutes und des Zitratplasmas innerhalb von 2-3 Stunden nach Blutabnahme erfolgt. Andererseits kann das Blut oder Plasma im Kühlschrank bis zu 24 h gelagert werden.

Bei dem erfindungsgemäßen Verfahren wird eine definierte Menge Thrombinhemmstoff in einer Blut- oder Plasmaprobe vorgelegt, worauf in dieser Probe die Gerinnung mittels intrinsischer und/oder extrinsischer Aktivatoren und optimaler Calciumionenkonzentration maximal induziert wird. Alles generierbare über die extrinsische und/oder intrinsische plasmatische Gerinnungsstrecke induzierte Gerinnungspotenzial setzt letztendlich die finale Serinprotease-Thrombin aus der inaktiven Vorstufe Prothrombin um. Das im Prothrombinasekomplex generierte und daraus freigesetzte Thrombin wird sofort von dem hochspezifischen Hemmstoff abgefangen und inaktiviert. Nach einer definierten Aktivierungszeit wird der Aktivierungsvorgang mittels EDTA gestoppt. Danach kann der nicht verbrauchte freie Hemmstoff in der Probe mittels einer präzisen, effizienten Bestimmungsmethode gemessen werden. Je mehr Thrombin generiert wurde, umso größer ist der Verbrauch an Inhibitor in der Probe. Damit ist es möglich, sehr präzise das maximale Thrombinaktivierungspotenzial einer Blut- oder Plasmaprobe zu erfassen. Der vorgelegte Hemmstoff bindet ausschließlich das permanent aktivierte Thrombin, sozusagen nur das Endprodukt der Gerinnung, und beeinflusst die davor ablaufenden Gerinnungsvorgänge in keiner Weise. Es ist bekannt, dass die im normalen Gerinnungsgeschehen durch Thrombin aktivierten Co-Enzyme FVa, FVIIIa auch durch den FXa aktiviert werden, so dass das reale physiologische bzw. pathologische Gerinnungsgeschehen bei dem erfindungsgemäßen Verfahren nachvollzogen wird.

Bei dem erfindungsgemäßen Verfahren werden die beiden bisher bekannten Aktivierungswege des Thrombins, der sogenannte extrinsische und der intrinsische Weg, gemeinsam berücksichtigt. Eine isolierte Aktivierung des extrinsischen oder intrinsischen Wegs ist möglich, aber nur in den Zellen von Bedeutung, bei denen Hinweise auf einen isolierten Faktomangel (z.B. durch angeborene Gerinnungskrankheiten) vorliegen. Ansonsten ist der Test ein "Globaltest" zur Entscheidung "gerinnungskrank" oder "gerinnungsgesund". Dementsprechend liegen Normwerte des erfindungsgemäßen Verfahrens im Blut wie auch im Plasma vor. Davon abweichende Werte bei einzelnen Patienten geben Hinweise auf eine Gerinnungskrankheit. Dabei wurde überraschenderweise gefunden, dass dabei ein optimales Mischungsverhältnis beider Aktivatorsubstanzen in einer optimalen Aktivierungsrezeptur eingesetzt werden müssen. Nur dadurch kann die maximal mögliche Thrombingenerierbarkeit gemessen werden. Hier können prinzipiell bekannte und auf dem Markt erhältliche Substanzen zur Gerinnungsinduktion verwendet werden. Extrinsische Aktivatoren sind üblicherweise Gewebefaktoren, die aus den verschiedensten Zell/Organkomponenten von Tieren und von Menschen hergestellt sind. Sogenannte "Thrornboplastine" oder "Thrombokinasen" werden aus Kaninchengehirn, aus Lunge oder Leber hergestellt und sind in der Regel schlecht standardisierbar. Es hat sich deshalb als vorteilhaft herausgestellt, ein rekombinantes Produkt zu benutzen, bei denen die Chargen keine größeren Aktivitätsschwankungen aufweisen. Entsprechendes gilt auch für die intrinsischen Aktivatoren. Hier sind sowohl tierische als auch pflanzliche Phospholipide benutzbar, die dann noch Starter wie Ellagsäure zusätzlich beinhalten. Entsprechende Produkte sind auf dem Markt erhältlich und einem Fachmann gut bekannt. Erfindungsgemäß wurde festgestellt, dass eine Kombination der Produkte Innovin und Actin FS (beides Produkte der Firma Dade Behring) die beiden Aktivierungswege des Thrombins in optimaler Weise berücksichtigen. Das Innovin ist ein rekombinantes Gewebefaktorpräparat und zielt auf den exogenen Aktivierungsweg ab. Das für den endogenen Aktivierungsweg benötigte Actin FS ist ein sogenanntes aktiviertes PTT-Reagenz, bei dem sowohl Phospholipide als auch Ellagsäure für die erforderliche endogene Gerinnungsaktivierung sorgen. Der Versuchsansatz sollte auch eine bestimmte optimale Menge an Calciumionen enthalten, damit das zunächst mit Citrat antikoagulierte Blut wieder neutralisiert wird und dass die Gerinnungsfaktoren, die in vivo das bluteigene Calcium zu ihrer Aktivierung benötigen, unter optimalen ionalen Verhältnissen vorliegen. Ferner sollte in dem Versuchsansatz Albumin (bevorzugt bovines Albumin) vorhanden sein, das der besseren Homogenisierung der lipidartigen Substanzen, vor allem des endogenen Aktivierungsweges dient. Für das erfindungsgemäße Verfahren wurde die Trivialbezeichnung THROGA (<u>thro</u>mbin <u>g</u>eneration <u>a</u>ssay) gewählt.

Die Erfindung betrifft somit auch ein Kit zur Bestimmung der Gesamtgerinnungsaktivität einer Blutprobe. Dieses Kit umfasst den Thrombinhemmstoff, Faktoren zur extrinsischen und intrinsischen Aktivierung des Thrombins sowie geeignete Hilfsreagenzien. Das Kit wird in an sich bekannten und auf übliche Weise herstellbaren Verpackungseinheiten bereitgestellt.

Mit dem erfindungsgemäßen Verfahren bzw. mit dem erfindungsgemäßen Kit kann gezielt und zuverlässig die maximal aktivierbare Thrombinmenge einer Blut- oder Plasmaprobe gemessen werden. Daneben kann mit dieser Methode auch eine basale Thrombinmenge im Blut gemessen werden. Im Plasma ist immer eine konstante kleine Menge von Thrombin zu finden. Im Blut jedoch sind unterschiedliche, sowohl verminderte als auch erhöhte Konzentrationen von Thrombin nachzuweisen ("blind thrombin").

Das erfindungsgemäße Verfahren gibt somit Auskunft über eine normale, übernormale oder unternormale Gerinnbarkeit des Blutes (Thrombophilie bzw. Hämophilie, Blutungstendenz). Ferner kann mit dem erfindungsgemäßen Verfahren eine Verlaufskontrolle einer Therapie mit oralen Antikoagulanzien vom Cumarin- oder Dicumaroltyp gemessen werden. Gegenüber dem bisher hierfür verwendeten Quickwert hat das erfindungsgemäße Verfahren (das THROGA-Verfahren) den Vorteil, dass orale Antikoagulanzien in ihrer Gerinnungshemmpotenz in Vollblut erfasst werden können. Durch das Verfahren kann beim Patienten somit eine Blutungstendenz frühzeitig erkannt werden. In Untersuchungen konnten gute Messergebnisse für die Patienten erhalten werden, die mit oralen Antikoagulanzien behandelt werden. Da bei der oralen Antikoagulationstherapie die Vorstufen der wesentlichen Serinproteasen des Gerinnungssystems in inaktiver Form vorliegen, vor allem die Vorstufe des Schlüsselenzyms Thrombin, das Prothrombin, ist es möglich, direkt das noch vorhandene Gerinnungspotenzial von derartigen Patienten zu bestimmen und durch die Erfassung der Thrombingenerierung im Blut auch Aussagen darüber zu erhalten, ob eine Blutungstendenz vorliegt. Ferner kann mit dem erfindungsgemäßen Verfahren auch eine Langzeitüberwachung einer Therapie mit anderen gerinnungshemmenden Pharmaka vorgenommen werden. In Versuchen zeigte sich, dass Patienten, die mit direkten Antithrombinsubstanzen wie Refludan oder Melagatran oder Argatroban behandelt werden, in ihrem thrombophilen Zustand überwacht werden können. Bei dem erfindungsgemäßen Verfahren handelt es sich somit um ein Akutverfahren, dass sofortige Aussagen zum komplexen Gerinnungsstatus eines Patienten erlaubt. Mit dem erfindungsgemäßen Verfahren können Unregelmäßigkeiten im Gerinnungsstatus des Blutes sofort erkannt werden, was die rasche Einleitung entsprechender Therapieschritte erlaubt.

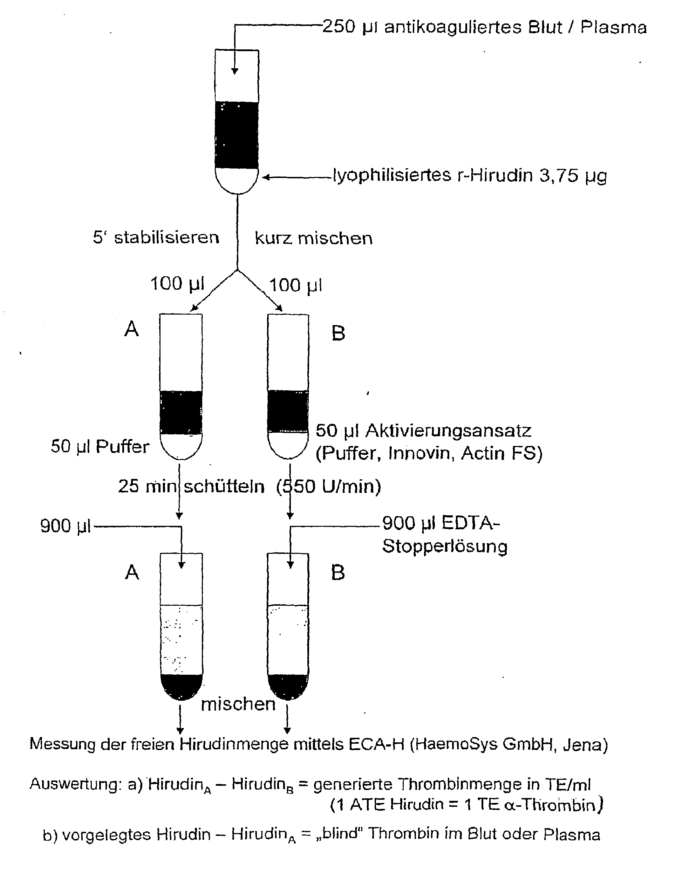

Die Figur 1 zeigt das erfindungsgemäße Verfahren im Überlick.

Die Erfindung wird anhand der nachstehenden Beispiele näher erläutert.

**Beispiel 1**

Allgemeine Vorschrift zur Testdurchführung

Zur maximalen Thrombingenerierung wird eine kleine Menge von antikoaguliertem Blut oder Plasma verwendet. Dazu kann Citrat, aber auch jedes andere Antikoagulans benutzt werden. Je 0,1 ml Citratblut oder -plasma werden in ein Referenz (NaCl)- und Aktivatorröhrchen (Gewebefaktor + Gemisch aus Eliagsäure und Phospholipiden) gegeben, in denen sich einen definierte Hirudinmenge befindet. Nach Verschluss der Reaktionsgefäße werden die beiden Gefäße in einem Minishaker bei 550 U/min bei Raumtemperatur 25 min lang gemischt. Die Gerinnungsaktivierung wird anschließend mit einer EDTA-haltigen Stopperlösung beendet und das restliche, nicht verbrauchte, freie Hirudin mit einer präzisen Nachweismethode bestimmt. Zur exakten Messung des Hirudingehalts in den Reaktionsgefäßen wird eine kommerzielle verfügbare chromogene Hirudinbestimmungsmethode mittels chromogenem Substrat (ECA, Ecarin chromogenic assay, WO 0046602) eingesetzt.

**Beispiel 2**

Beispiel eines THROGA-Kits

Nachstehend ist die beispielhafte Zusammensetzung eines THROGA-Kits angegeben:

25 x   Aktivator-Röhrchen mit lyophilisiertem Reagenz, Kennzeichnung: rote Markierung

25 x   Referenz-Röhrchen mit lyophilisiertem Reagenz

1 x   Referenz-Röhrchen mit lyophilisiertem Reagenz für die Referenzkurve, Kennzeichnung: Verschluss mit rotem Punkt

1 x   10fach konzentriertes Stopp-Reagenz, 6 ml

2 x   THROGA-Kontrolle, lyophilisiert, für 1,5 ml

2 x   ECA-Prothrombin-Puffer, lyophilisiert, für 6 ml

1 x   ECA-H-Substrat, gebrauchfertige Lösung, 3 ml

2 x   ECA-Ecarin-Reagenz, lyophilisiert, für 3 ml.

ECA-Prothrombin-Puffer, ECA-H-Substrat, ECA-Ecarin-Reagenz: 7 Tage im Gebrauch
(getestet durch tägliche Temperierung bei 37°C für 3h, Lagerung zwischen den Messungen bei 2-8°C)

ECA-Prothrombin-Puffer, ECA-H-Substrat, ECA-Ecarin-Reagenz, portioniert und verschlossen gelagert bei 2-8°C: 28 Tage

Bei Aufbewahrung der Reagenzien bei 37°C im Messgerät sind die vials zum Schutz vor Verdunstung nach jeder Reagenzienentnahme zu verschließen. Nach Beendigung der Arbeiten sind die Reagenzien verschlossen bei 2-8°C aufzubewahren.

**Beispiel 3**

Durchführung der Bestimmung der maximalen Thrombingeneration in Citratblut und/oder Plasma

Vorbereitung der Reagenzien

Der voreingestellte Schüttler muss mindestens 30 Minuten bevor die Probenröhrchen hineingestellt werden, eingeschaltet werden. Das 10fach konzentrierte Stopp-Reagenz ist, je nach benötigter Menge, 1:10 mit destilliertem Wasser zu verdünnen. Das ECA-H-Substrat ist gebrauchsfertig. ECA-Ecarin-Reagenz, ECA-Prothrombin-Puffer und THROGA-Kontrolle werden mit der auf dem Etikett angegebenen Menge destilliertem Wasser aufgelöst und durch Über-Kopf-Drehen gut aber vorsichtig gemischt. Die Reagenzien und die Kontrolle müssen 45min bei RT rekonstituiert werden. Mindestens 1x während und nach dieser Rekonstitutionszeit werden die Reagenzien durch Über-Kopf-Drehen vorsichtig gemischt.

**Erforderliche aber nicht enthaltene Materialien und Geräte**

- manuelles Gerinnungsmessgerät mit Option zur Messung chromogener Reaktionen Coatron M2 adaptiert auf ECA-H, einschließlich Verbrauchsmaterialien und Bedienungshandbuch (Informationen zum Bezug bei HaemoSys GmbH)

- voreingestellter Schüttler (ca. 500 U/min)

- Stoppuhr

- entionisiertes oder destilliertes Wasser

- geeignete kalibrierte Pipetten mit Spitzen

- Blutprobenentnahmematerialien

**Spezimen/Untersuchungsmaterial**

- Citratplasma aus Citratblut, möglichst rasch mindestens 10min bei 1500xg zentrifugieren und Plasma separieren.

- Citratblut mit einem Verhältnis von 1 Teil Natriumcitratlösung (0,11mol/l) mit 9 Teilen Venenblut sorgfältig unter Vermeidung von Schaumbildung mischen.

- Stabilität der Probe bei 15-25°C: 4h

**Methodik**

Thrombingenerierung

Für eine Bestimmung werden je in ein Aktivator-Röhrchen und ein Referenz-Röhrchen 100µl Citrat-blut bzw Citratplasma gegeben. Um Verdunstung zu vermeiden, sollten die Röhrchen nach dem Lösen verschlossen werden. Die Röhrchen werden anschließend für 30 - 60 Minuten in den Schüttler gestellt. Um die Reaktion nach dem Schütteln zu beenden, wird in jedes Röhrchen 1000µl Stopp-Reagenz zugegeben und mit der Pipette vorsichtig durchmischt.

| Refenz-Röhrchen | Aktivator-Rohrchen |
|---|---|
| 100µl Plasma | 100µl Plasma |
| 30min Schütteln ||
| 1000µl Stopp-Reagenz | 1000µl Stopp-Reagenz |

Bestimmung der Anti-Thrombin Einheiten (A-T-E) mittels ECA-H

In beiden THROGA-Proben (Referenz- und Aktivator-Röhrchen) werden nun die Anti-Thrombin-Einheiten (A-T-E) mittels ECA-H bestimmt.

ECA-H kann mit einer Vielzahl von automatischen und manuellen Koagulometern, die mit einer Option zur optischen Messung ausgestattet sind, verwendet werden.

Testdurchführung der Methode unter Verwendung des auf ECA-H adaptierten Gerinnunosmessgerätes Coatron M2

Es ist die Bedienungsanleitung des Coatron M2 zu beachten. Bei der Bestimmung des THROGA in Blutproben muss die Option "Autostart" im Gerät inaktiviert werden.

Das Messgerät wird auf 37°C vorgewärmt. Die Küvetten werden im Küvettenblock des Messgerätes vorgewärmt. ECA-Prothrombin-Puffer, ECA-Ecarin-Reagenz und, wenn möglich, das ECA-H-Substrat werden im Gerät mindestens 15min vorgewärmt.

Zur Vermeidung von Verdunstung sollten die Reagenzien während der Messung verschlossen oder abgedeckt werden.

Detaillierte Informationen zur Testdurchführung am Coatron M2 sind dem Anhang zur Bedienungsanleitung (Applikation ECA-H) zu entnehmen.

Reagenzien und THROGA-Probe werden entsprechend dem Pipettierschema in die Küvetten pipettiert.

Pipettierschema: ECA-H

| In auf 37°C vorgewärmte Küvetten pipettieren | |
|---|---|
| ECA-Prothrombin-Puffer | 100µl |
| THROGA-Probe | 25µl |
| ECA-H-Substrat | 25µl |
| mischen, 1min Inkubation bei 37°C | |
| ECA-Ecarin-Reagenz | 50 µl |
| Wenn die Zugabe des ECA-Ecarin-Reagenzes nicht mit einer Starterpipette erfolgt, wird die Zeitmessung automatisch 15s nach der Reagenzzugabe gestartet. | |

Auswertung

Das Messergebnis wird als Messzeit in Sekunden und als U (Anti-Thrombin-Einheiten, A-T-E) ausgegeben. Die Auswertung erfolgt über eine im Messgerät gespeicherte Referenzkurve.

Aus den ermittelten A-T-E in Referenz- bzw. Aktivator-Röhrchen werden die in der Plasma- oder Blutprobe gebildeten Thrombin-Einheiten mit folgender Formel berechnet:

$$ETP = R - A$$

ETP    maximales endogenes Thrombinpotential, in 1ml Blut oder Plasma gebildete Thrombin-Einheiten

R    Anti-Thrombin-Einheiten im Referenz-Röhrchen (A-T-E/ml Blut oder Plasma)

A    Anti-Thrombin-Einheiten im Aktivator-Röhrchen (A-T-E/ml Blut oder Plasma)

Erstellung einer Referenzkurve

Um korrekte Ergebnisse zu erhalten, muss für jede neue Reagenziencharge eine Referenzkurve erstellt werden. Zur Erstellung der Referenzkurve werden anstelle der THROGA-Probe je 25µl THROGA-Standard eingesetzt. Es wird eine 4-Punkt Messung durchgeführt:

Herstellung der THROGA-Standard Verdünnungen:

| THROGA-Standard (Konzentration in Anti-Thrombin Einheiten / ml Plasma) | Herstellung |
|---|---|
| 224 | Referenz-Röhrchen gelöst in 1100 μl Stopp-Reagenz |
| 112 | 500 μl Standard 225 A-T-E + 500 μl Stopp-Reagenz |
| 56 | 500 μl Standard 112,5 A-T-E + 500 μl Stopp-Reagenz |
| 0 | Stopp-Reagenz |

Es wird empfohlen, die Messung der THROGA-Standards als Doppelbestimmung durchzuführen. Es werden die Mittelwerte der aus den Messungen erhaltenen ECA-H-Reaktionszeiten gebildet und mit den entsprechenden Standard-Konzentrationen in das Messgerät eingegeben. Dabei ist zu beachten, dass U der Konzentrationseinheit **A-T-E**/ml Blut oder Plasma entspricht. Die Referenzkurve wird im Messgerät gespeichert.

Detaillierte Informationen zur Erstellung von Referenzkurven am Coatron M2 sind dem Anhang zur Bedienungsanleitung der Messgeräte (Applikation ECA-H) zu entnehmen.

Interne Qualitätskontrolle

Für die interne Qualitätskontrolle wird die vorbereitete THROGA-Kontrolle anstelle von Blut oder Plasma im THROGA eingesetzt

Liegt der gemessene Kontrollwert außerhalb des auf dem Etikett der THROGA-Kontrolle angegebenen Kontrollbereiches, sind keine zuverlässigen Bestimmungen gewährleistet. Die Reagenzien sollten überprüft und gegebenenfalls erneuert werden. Liegt der gemessene Kontrollwert weiterhin außerhalb des Kontrollbereiches, ist die Funktionsfähigkeit des Messgerätes zu überprüfen.

Beispiel 4

Untersuchung von Patienten mittels THROGA:
a) Probanden: I.N., weiblich, 37 Jahre

| | r-Hirudin-Konzentration (μg/ml) | 1 μg = 15,8 ATE |
|---|---|---|
| | Blut | Plasma |
| Kontrolle (A) | 14,9 | 14,6 |
| Aktivierte Probe (B) | 9,3 | 7,5 |
| Differenz (μg/ml) | 5,6 | 7,1 |
| ATE/ml; TE/ml | **88,5** | **112,2** |
| Auswertung: 1.N. hat im Plasma eine "normale" Aktivierungsmenge von 112,2 (N: 122±19 TE/ml) | | |

Im Blut der Probandin sind 88,5 TE/ml generiertes Thrombin gemessen worden. Der Normwert liegt bei 78±13 TE/ml. Damit liegt sie ebenfalls in dem Normbereich.
b) Patienten: C.R., weiblich, 55 Jahre, Zustand nach zerebralem Insult

1. vor Beginn einer Blutplättchen-spezifischen Therapie mit Clopidogrel (7.1.04)

| | r-Hirudin-Konzentration (μg/ml) | 1 μg = 15,8 ATE |
|---|---|---|
| | Blut | Plasma |
| Kontrolle (A) | 13,2 | 14,2 |
| Aktivierte Probe (B) | 6,9 | 6,7 |

(fortgesetzt)

| | r-Hirudin-Konzentration (μg/ml) | 1 μg = 15,8 ATE |
|---|---|---|
| | Blut | Plasma |
| Differenz (μg/ml) | 6,3 | 7,5 |
| ATE/ml; TE/ml | **99,5** | **118,5** |

2. nach 8 Wochen Behandlung mit Clopidogrel tägl. 1,5 Tabl. (112,5 mg), (4.3.04)

| | r-Hirudin-Konzentration (μg/ml) | 1 μg = 15,8 ATE |
|---|---|---|
| | Blut | Plasma |
| Kontrolle (A) | 14,1 | 14,3 |
| Aktivierte Probe (B) | 8,9 | 6,9 |
| Differenz (μg/ml) | 5,2 | 7,4 |
| ATE/ml; TE/ml | **82,2** | **116,9** |

Auswertung: Pat. C.R. hatte bei Erstuntersuchung einen pathologischen THROGA-Wert im Blut von 99,5 (Norm: $78 \pm 13$ TE/ml), der sich nach 8 Wochen plättchenspezifischer Therapie normalisiert hat (82,2). Die THROGA-Plasmawerte waren bei beiden Untersuchungen im Normbereich! Bei allen weiteren Kontrollen unter Clopidogrel-Therapie waren die THROGA-Werte normal.

c) Patient M.F., männlich, 64 Jahre, Diagnose: Thrombophilie bei Gammopathie; Pat. Erhält Thromboseprophylaxe mit Falithrom (orales Antikoagulans), 12.2.05

Verwendung des Kits:

| | Blut (ATE/ml) | Plasma (ATE/ml) |
|---|---|---|
| Kontrolle (A) | 265,4 | 257,5 |
| Aktivierte Probe (B) | 219,1 | 218,2 |
| Differenz (μg/ml) | **46,3** | **39,3** |

Auswertung: Pat. M.F. hat mit 39,3 TE/ml im Plasma 32% des normalen Plasmawertes und ist somit in einem ausreichenden "therapeutischen Fenster". Im Blut wurden bei 46,3 TE/ml noch 60% der normalen generierbaren Thrombinmenge nachgewiesen. Damit ist keine Blutungstendenz festzustellen und dieser Patient ist optimal versorgt.

**Patentansprüche**

1. Verfahren zur Bestimmung der Gesamtgerinnungsaktivität einer Blut- oder Plasmaprobe zur Entscheidung über "gerinnungskrank" oder "gerinnungsgesund", **dadurch gekennzeichnet, dass** man einen hochspezifischen, nicht irreversiblen Thrombinhemmstoff, ausgewählt aus Dipetalogastin I, Dipetalogastin II, Rhodniin und Hirudin, in definierter Menge einer Blut- oder Plasmaprobe zusetzt, die Gerinnung in der Blut- oder Plasmaprobe durch Zusatz von extrinsischen und intrinsischen Faktoren induziert und nach einer definierten Zeit die verbrauchte Menge des zugesetzten Thrombinhemmstoffs in an sich bekannter Weise bestimmt.

2. Verfahren nach Anspruch 1, **dadurch gekennzeichnet, dass** die Gerinnungsinduktion durch einen Gewebefaktor und ein Gemisch aus Ellagsäure und Phospholipiden erzeugt wird.

3. Kit zur Verwendung zur Bestimmung der Gesamtgerinnungsaktivität einer Blutprobe zur Entscheidung über "gerinnungskrank" oder "gerinnungsgesund", **dadurch gekennzeichnet, dass** es umfasst

i) einen hochspezifischen, nicht irreversiblen Thrombinhemmstoff, ausgewählt aus Dipetalogastin I, Dipetalogastin II, Rodniin und Hirudin,
ii) Faktoren zur extrinsischen und intrinsischen Aktivierung des Thrombins,
iii) erforderliche Reagenzien und Hilfsstoffe.

**4.** Verwendung eines Kits nach Anspruch 3 zum Erhalt von Aussagen über den Gerinnungsstatus einer Blutprobe.


**Claims**

**1.** A method for determining the overall coagulation activity of a blood or plasma sample so as to decide whether it is "coagulation-disordered" or "coagulation-sound", **characterised in that** a highly specific, non-irreversible thrombin inhibitor selected from dipetalogastin I, dipetalogastin II, rhodniin and hirudin is added to a blood or plasma sample in a defined amount, coagulation in the blood or plasma sample is induced by the addition of extrinsic and intrinsic factors and the amount of the added thrombin inhibitor consumed after a defined time span is determined in a manner known per se.

**2.** A method according to claim 1, **characterised in that** the induction of coagulation is triggered by a tissue factor and a mixture of ellagic acid and phospholipids.

**3.** A kit for use in the determination of the overall coagulation activity of a blood sample so as to decide whether it is "coagulation-disordered" or "coagulation-sound", **characterised in that** it comprises

i) a highly specific, non-irreversible thrombin inhibitor selected from dipetalogastin I, dipetalogastin II, rhodniin and hirudin,
ii) factors for the extrinsic and intrinsic activation of the thrombin,
iii) reagents and adjuvants as required.

**4.** The use of a kit according to claim 3 for obtaining information on the coagulation status of a blood sample.


**Revendications**

**1.** Procédé pour déterminer l'activité de coagulation totale d'un échantillon de sang et de plasma servant à une décision « malade concernant la coagulation » ou « saine concernant la coagulation », **caractérisé en ce qu'**on ajoute en quantité définie un inhibiteur non irréversible et hautement spécifique de la thrombine à un échantillon de sang ou de plasma, l'inhibiteur étant choisi parmi la dipetalogastine I, la dipetalogastine II, la rhodniine et l'hirudine,
**en ce qu'**on induit la coagulation dans l'échantillon de sang ou de plasma par l'addition des facteurs intrinsèques ou extrinsèques et
**en ce qu'**on détermine de manière connue en soi la quantité utilisée de l'inhibiteur de thrombine ajouté après une durée définie.

**2.** Procédé selon la revendication 1, **caractérisé en ce que** l'induction de la coagulation est faite avec un facteur de tissue et un mélange de l'acide ellagique et des phospholipides.

**3.** Nécessaire servant pour la détermination de l'activité de coagulation totale d'un échantillon de sang ou de plasma servant à une décision « malade concernant la coagulation » ou « saine concernant la coagulation », **caractérisé en ce qu'**il comprend

i) un inhibiteur non irréversible et hautement spécifique de la thrombine, choisi parmi la dipetalogastine I, la dipetalogastine II, la rhodniine et l'hirudine,
ii) des facteurs pour l'activation intrinsèque et extrinsèque de la thrombine,
iii) des réactifs nécessaires et des adjuvants nécessaires.

**4.** Utilisation d'un nécessaire selon la revendication 3 pour obtenir des indications sur l'état de coagulation d'un échantillon sanguin.

250 µl antikoaguliertes Blut / Plasma

lyophilisiertes r-Hirudin 3,75 µg

5' stabilisieren  kurz mischen

100 µl  100 µl

A  B

50 µl Puffer  50 µl Aktivierungsansatz (Puffer, Innovin, Actin FS)

25 min schütteln (550 U/min)

900 µl  900 µl EDTA-Stopperlösung

A  B

mischen

Messung der freien Hirudinmenge mittels ECA-H (HaemoSys GmbH, Jena)

Auswertung: a) Hirudin$_A$ – Hirudin$_B$ = generierte Thrombinmenge in TE/ml
(1 ATE Hirudin = 1 TE α-Thrombin)

b) vorgelegtes Hirudin – Hirudin$_A$ = „blind" Thrombin im Blut oder Plasma

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0420332 A, Hemker
- WO 0052199 A
- WO 03093831 A
- WO 09621740 A

- EP 1367135 A
- US 6051390 A
- WO 0046602 A

**In der Beschreibung aufgeführte Nicht-Patentliteratur**

- Die quantitative Bestimmung des Prothrombins durch Titration mit Hirudin. *Arch. exper. Path. u. Pharmakol.,* 1958, vol. 232, 487-498